# EUROPÄISCHE PATENTANMELDUNG

(11) **EP 1 527 776 A1**
(43) Veröffentlichungstag der Anmeldung: **04.05.2005**
(21) Anmeldenummer: 04025873.3
(22) Anmeldetag: 01.11.2004
(51) Int. Cl.: A61K 31/16, A61K 31/215, A61K 31/557, A61P 43/00, A61P 29/00, A61P 1/00, A61P 9/12, A61P 11/06, A61P 25/00, A61P 25/02, A61P 25/06, A61P 25/18, A61P 27/02, A61P 37/06

(54) **Arzneimittel (amid oder Ester einer Omega-polyensäure und einem biogenen Amin oder Alkohol) und Verfahren zu ihrer Herstellung**

(30) Priorität: 03.11.2003 DE 10351111
(71) Anmelder: Langlotz, Rainer, Dipl.-Chem., 68804 Altlussheim (DE)
(72) Erfinder: Langlotz, Rainer, Dipl.-Chem., 68804 Altlussheim (DE)
(74) Vertreter: Hermann, Wolf-Dieter

(57) **Zusammenfassung**

Arzneimittel, enthaltend eine Fettsäureverbindung der Formel R-CO-Y mit R als Omega-x-Polyensäurerest mit x=3-9 und einer Gesamtkohlenstoffanzahl zwischen C₁₇ und C₂₃ sowie einer Anzahl an isolierten Doppelbindungen zwischen 3 und 6 (z.B. Gammalinolensäure, Arachidonsäure, Omega-3-Docosahexaensäure).
Y kann entweder ein biogenes Amin der Struktur -NH-R' oder ein einfaches Derivat davon bzw. ein biogener Alkohol der Struktur -O-R' oder ein einfaches Derivat davon sein.
Das biogene Amin ist z.B. eine decarboxylierte natürliche Aminosäure AS oder ein einfaches Derivat davon aus der Reihe Val, Ileu, Leu, Pro, Hypro, Arg, CyS-SCy, Met, Lys, Hylys, Orn, Trp, His. Als Wirksubstanz wird entsprechend ein Fettsäureamid ausgebildet.
Der biogene Alkohol ist von einer decarboxylierten natürliche Aminosäure abgeleitet aus der Reihe Ala, Val, Ileu, Leu, Phe, Hypro, Arg, Thr, Cys, CyS-SCy, Met, Lys, Hylys, Orn, Trp und His. Als Wirksubstanz wird entsprechend ein Fettsäureester ausgebildet.
Das Arzneimittel dient als aktive Wirksubstanz zur Bekämpfung von Krankheiten und Störungen wie z.B. Depression, Angst, Schmerz, Krampflösung, Erbrechen, Bluthochdruck, Dyskinesie, Schlaflosigkeit, Migräne, Asthma, Schizophrenie, multipler Sklerose, Appetitanregung.

## Beschreibung

Die Erfindung betrifft neue, pharmazeutisch aktive chemische Verbindungen, die im Endocannabinoidsystem des Menschen Wirkung entfalten.

Hochungesättigte Fettsäuren mit 3 und mehr Doppelbindungen, insbesondere die so genannten Omega-3 und Omega-6 Fettsäuren haben in den letzten Jahren verstärkt das Interesse der Pharmakologen erregt. Dabei bezeichnet "Omega-x" in diesem Zusammenhang die Position der ersten Doppelbindung vom freien Alkyl- Ende der Fettsäurekette aus betrachtet. In Naturprodukten finden sich häufig Omega-3 bis Omega-6 Fettsäuren. Diese Fettsäuren sind als Ester Bestandteile zahlreicher Nahrungsmittel und nehmen Schlüsselpositionen bei einer Vielzahl von physiologischen Abläufen ein. Als Ausgangspunkt für eine ganze Kaskade von hormonähnlichen Substanzen erwies sich vor allem die Omega-6-Eicosatetraensäure, die man gewöhnlich als Arachidonsäure bezeichnet. Arachidonsäure entsteht im Körper aus ungesättigten Fettsäuren der Nahrung über Gammalinolensäure und Dihomogammalinolensäure als Zwischenstufen. Arachidonsäure stellt die Ausgangsverbindung für die Synthese von Prostaglandinen, Leukotrienen, Thromboxanen, und Lipoxinen im Körper dar. Auch die verwandte Dihomogammalinolensäure sowie die Eicosapentaensäure sind Ausgangssubstanzen für die in vivo Synthese von Prostaglandinen und anderen Gewebshormonen. Die meisten dieser hormonähnlichen Produkte zeichnen sich durch eine Vielzahl von physiologischen Aktivitäten bei allerdings geringer Wirkdauer aus. Dies verhindert zumeist den direkten therapeutischen Einsatz solcher Gewebshormone. Man versucht stattdessen ihre Konzentration im Körper zu beeinflussen, indem man ihre enzymatische Synthese fördert bzw. ihren Abbau blockiert. "Eicosanoide", wie man diese ganze von Arachidonsäure abgeleitete Substanzklasse nennt, beeinflussen Blutdruck, Blutgerinnung, fördern oder hemmen Entzündungen, beeinflussen allergische Reaktionen und die Aktivität des Immunsystems. Bereits diese Vielfalt der Anwendungsgebiete macht die Eicosanoide zu einer weitreichenden Entdeckung in der Humanphysiolgie der letzten 3 Jahrzehnte.

Innerhalb der Eicosanoide ist Arachidonsäure-2-hydroxyethylamid (AEA), als Vertreter einer neuen Verbindungsklasse bekannt geworden. [1]. Im Jahre 1992 wurde AEA als erster körpereigener Agonist des CB-1 Rezeptors entdeckt; jener Schaltstelle, die durch Delta-9-THC - dem psychoaktiven Molekül der Cannabispflanze - aktiviert wird. Detaillierte Studien der folgenden Jahre zeigten, dass auch Hydroxyethylamide anderer hochungesättigter Fettsäuren, wie Eicosapentaensäure und Docosatetraensäure diesen Cannabinoidrezeptor aktivieren.
Der Fettsäurerest in der Biomolekel spielt eine bedeutende Rolle beim Wirkmechanismus der neuen Arzneimittel in vivo. Die Doppelbindungen aller bekannten Fettsäuren, die am Cannabinoidrezeptor CB-1 wirken, besitzen ausschließlich Z-Konformation und sind durch je eine Methylengruppe voneinander getrennt. Ferner ist bekannt, dass die Polyensäurereste in "Haarnadel"-Konformation vorliegen und als solche am aktiven Zentrum des Rezeptors angreifen. In früheren Arbeiten hat sich gezeigt, dass insbesondere von der Arachidonsäure, der Eicosapentaensäure, der Docosatetraensäure sowie der Docosapentaensäure abgeleitete Verbindungen pharmakologische Wirkungen am CB-1 Rezeptor entfalten [2]. 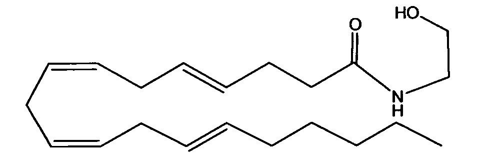 Arachidonylethanolamid (AEA) in Haarnadelkonformation

Es zeigte sich jedoch auch, dass die Hydroxyethylamide nur Partialagonisten an diesem Rezeptor sind und daher nur schwach ausgeprägte pharmakologische Wirkungen aufweisen. Diese "klassischen" endogenen Cannabinoide * weisen indes erhebliche Nachteile auf, denn gezielte und nachhaltige pharmakologische Wirkungen lassen sich mit der Stammsubstanz AEA und ihren bisher bekannten Analoga kaum erzielen.

* Der Begriff "Cannabinoid" bezieht sich entweder auf Verbindungen, welche physiologische Effekte ähnlich denen der Pflanze Cannabis sativa produzieren, oder allgemeiner auf Verbindungen, die direkt oder indirekt eine agonistische oder antagonistische Wirkung an den Cannabinoidrezeptoren CB-1 oder CB-2 entfalten können. Im Folgenden ist diese allgemeinere Definiton des Begriffs "Cannabinoid" gemeint.

Man führt diesen Befund vor allem auf den zu schnellen Abbau der einfachen AEA-Analoga durch das Enzym FAAH (Fatty acid amide hydrolase) zurück. So sind FAAHblockierende Pharmaka wie Indomethacin® und Ibuprofen® seit vielen Jahren als Schmerzmittel weit verbreitet. Bevor man den THC-Rezeptor kannte, also bevor man den Mechanismus ihrer Wirkung auch nur annähernd verstehen konnte, wurden diese Verbindungen rein empirisch gefunden.
Durch Variation der 2-Position hinter der Carboxylgruppe des Fettsäurerestes gelangt man zu Molekülen, die nur langsam abgebaut werden und deshalb längere und intensivere Wirkungen entfalten. Hier ragen besonders die 2'-Fluoro-2-methylanandamide hervor, welche "robuste pharmakologische Wirkungen" entfalten [3]. Der Einsatz solcher Arzneien am Menschen ist jedoch gewagt, da ein in vivo Abbau des Moleküls zu hochtoxischen Fluorcarbonsäuren nicht ausgeschlossen ist.

Im Jahre 1995 wurde ein weiteres endogenes Cannabinoid entdeckt, das 2-Glyceroylarachidonat (2-GA), ein Ester der Arachidonsäure mit Glycerin [4]. Es ist im Gehirn von Säugetieren um 2 Größenordnungen häufiger zu finden als AEA. Diese Stoffgruppe wirkt im Vergleich zu den Hydroxyethylamiden stärker analgetisch, etwas euphorisierend aber zugleich auch sedativ. Allerdings wird auch das 2-GA zu schnell abgebaut, um pharmakologisch oder psychopharmakologisch nutzbringend eingesetzt zu werden. Deshalb können auch diese Strukturen die Forderungen moderner Pharmakologie hinsichtlich Intensität und Wirkdauer eines Arzneimittels nicht befriedigen.
Durch intensive Forschungsarbeit, die auf die Entdeckung der endogenen Cannabinoide folgte, wurde zudem gefunden, dass neben dem CB-1 genannten Rezeptor im Zentralnervensystem auch periphere Rezeptoren für solche Substanzen vorhanden sind, welche als CB-2 Rezeptoren bezeichnet werden. [5].
Neuerdings wurde gefunden, dass das Gehirn wenigstens 5 Verbindungen produziert, welche sub-micromolare Affinität zu Cannabinoid Rezeptoren zeigen: Neben Arachidonsäure-2-hydroxyethylamid (AEA) und 2-Glyceroylarachidonat (2-GA), sind dies: Noladinether, Virodhamin, und N-Arachidonoyldopamin (NADA). Letztere Verbindung gilt neben AEA als hauptsächlicher endogener Agonist des mit dem Cannabinoidsystem eng verbundenen Vanilloidrezeptors (VR-1) [6]. Hier zeigt sich eine prinzipielle Problematik bei der Entwicklung selektiv wirksamer Pharmaka; nämlich die Tatsache, dass viele endogene oder synthetische Cannabinoide neben CB-1 und CB-2 Rezeptoren gleichzeitig auch am VR-1 Rezeptor aktiv sind. [7]
In einer weiteren Verbindungsklasse sind mit dem Fettsäurerest phosphorylierte Aminosäuren gekoppelt. Diese binden sich jedoch nur an peripheren CB-2 Rezeptoren, da sie nicht in der Lage sind die Bluthirnschranke nennenswert zu passieren. [8]
Es bleibt daher nach wie vor erstrebenswert, nach selektiv wirksamen und zugleich physiologisch unbedenklichen Cannabinoiden zu forschen, zumal das therapeutische Potential solcher Wirkstoffe ständig wächst. Bislang war es nämlich weder für Arachidonsäure noch für andere Omega-x-Fettsäuren gelungen, geeignete Partner zu finden, um zu gezielten Wirkstoffen zu gelangen, welche selektiv bestimmte Eigenschaften des Endocannabinoidsystems hervortreten lassen.

Demzufolge ist es unter Meidung der im Stand der Technik bekannten Nachteile Aufgabe vorliegender Erfindung, zur Bekämpfung von Depressionen, Angstzuständen, Schmerzen, Krämpfen, Erbrechen, Bluthochdruck, psychomotorischer Unruhe, Überagitiertheit, Schlaflosigkeit, bestimmten Formen von Schizophrenie, Chemotherapie-Nebenwirkungen sowie erhöhtem Augeninnendruck geeignete oder als Immunsupressivum, insbesondere bei Behandlung der multiplen Sklerose, oder als Neuroprotektivum bei Gehirntraumen oder als Inhibitor von "Tumor Necrosis Faktor" (TNF-alpha) oder bei peripheren vasculären Erkrankungen oder zur Behebung motorischer Störungen oder zur Reduzierung von L- DOPA- Dyskinesien (z.B. bei Parkinson-Patienten) oder zur Appetitanregung oder Appetitunterdrückung oder zur Förderung von Haarwuchs einsetzbare, physiologisch unbedenkliche, hochwirksame und nebenwirkungsfreie Arzneimittel, insbesondere in den Wirkstoffklassen der Psychopharmaka, Antidepressiva, Anxiolytika, Analgetika, Spasmolytika, Antiemetika, Sedativa, Antihypertensiva, Antiasthmatika, Migränemittel sowie der Immunsupressiva zur Verfügung zu stellen, welche nach enzymatischem Abbau in der Zelle ausschließlich körpereigene oder körperkompatible Metaboliten freisetzen. Diese sind als physiologisch unbedenklich bekannt und lösen daher keinerlei störende Nebenwirkungen aus.

Während die Auswirkungen von Variationen des Fettsäurerests cannabinoider Fettsäurederivate intensiv erforscht wurde, ist über Derivate mit anderen Amin bzw. Alkoholanteilen nur wenig berichtet worden [2]. Bei diesen Variationen hielt man sich eng an die Ethanolamin- Struktur des ursprünglich entdeckten Anandamids.

Erfindungsgemäß wurde gefunden, dass sich eine Vielzahl von "biogenen Aminen" bzw. "biogenen Alkoholen", d.h. decarboxylierte natürliche Aminosäuren bzw. deren Hydroxy-Analoga, zu pharmakologisch wirksamen Fettsäureamiden bzw. Fettsäurestern umsetzen lassen. Da solche biogenen Amine bzw. Alkohole -genau wie die freien Omega-x-Fettsäuren in jedem Organismus vorkommen, lösen diese auch keine merklichen Nebenwirkungen im Körper aus. Dennoch rufen diese bisher noch nicht bekannten Fettsäurederivate Wirkungen an Cannabinoid- Rezeptoren hervor.
Dabei besteht die aktive Wirksubstanz eines entsprechenden Arzneimittels aus drei Strukturteilen: dem Fettsäureanteil, der Carbonylgruppe, und der Seitenkette am Amid bzw. Esterheteroatom.
Weiterhin lassen sich von beiden biogenen Wirkstoffgruppen einfache Derivate ableiten, die sich für entsprechende Synthesen von Arzneimitteln eignen.

Es konnte vorliegend gezeigt werden, dass es sehr wohl möglich ist, selektive und hochwirksame "Cannabinoide" zu erhalten. Diese sind in den erforderlichen Dosierungen völlig unbedenklich. Aufgrund ihrer Verwandtschaft mit körpereigenen Substanzen besitzen solche Stoffe ein großes Potential als nebenwirkungsarme Medikamente in verschiedenen pharmakologischen Sektoren.
Potentielle Einsatzgebiete für die neu gefundenen Fettsäurederivate sind: Bekämpfung von Depressionen, Angstzuständen, Schmerzen, Krämpfen, Erbrechen, Bluthochdruck, psychomotorischer Unruhe, Überagitiertheit, Schlaflosigkeit, bestimmten Formen von Schizophrenie, Chemotherapie Nebenwirkungen, erhöhtem Augeninnendruck, als Immunsupressiva, insbesondere bei Behandlung multipler Sklerose, als Neuroprotektiva bei Gehirntraumen, als Inhibitoren von "Tumor Necrosis Faktor" (TNF-alpha), bei peripheren vascularen Erkrankungen, zur Appetitanregung oder Unterdrückung und zur Förderung von Haarwuchs.

Man geht davon aus, dass sich die pharmakolgische Wirkung dieser Verbindungsklasse in der Zelle derart entfaltet, dass sich der lipophile Molekülteil am Rezeptor bindet. Dabei können solche Spezies sowohl am CB-1, CB-2 als auch am VR-1 Rezeptorsystem aktiv sein. [9] In pharmazeutischen Präparaten findet man dann - abhängig vom jeweiligen Partner am Omega-x-Polyensäurerest - starke agonistische oder antagonistische Wirkungen.

Der Erfindung liegt die Erkenntnis zugrunde, dass in Arzneimittel mit einer aktiven Struktur der Form R-CO-Y mit R als Omega-x-Polyensäurerest und Y als Amin oder Alkohol der Strukturen ―NH-R' bzw. ―O-R' *biogene* Reste R' eingeführt werden können. Diese biogenen Reste stammen aus natürlichen Aminosäuren ("AS"), die vor der Einführung decarboxyliert werden. Im Falle der biogenen Alkohole wird noch die Alpha-Aminogruppe durch die OH-Gruppe ersetzt. Es liegen schließlich biogene Amine oder biogene Alkohole vor oder deren Derivate, die mit dem entsprechenden mehrfach ungesättigten Fettsäurerest acyliert sind. Im Folgenden soll -NH-R' den biogenen Aminund -O-R'den biogenen Alkoholrest bezeichnen.

Aufbauend auf der Erkenntnis, dass sich der Aminrest des zunächst entdeckten Arachidonoylethanolamids von einer körpereigenen Aminosäure herleitet und der daraufhin erfolgten systematischen Erforschung von Fettsäureamiden der anderen körpereigenen, von Aminosäuren abgeleiteten Amine und Alkohole liegt die erfinderische Tätigkeit in der Lehre, dass auch diese im menschlichen Endocannabinoidsystem agonistische oder antagonistische Wirkungen entfalten. Dies ist bei den beträchtlichen chemischen und physiologischen Unterschieden innerhalb der Gruppe der biogenen Amine und Alkohole völlig überraschend.
Dass die biogenen Fettsäureamide bzw. Fettsäureester am Endocannabinoidsystem des Menschen eine allgemeine Aktivität entfalten, ist keineswegs naheliegend.

Die Erfindung erstreckt sich auf Amide bzw. Ester mit Fettsäureresten R, vorzugsweise aus 3-6 fach ungesättigten Fettsäuren der Kettenlänge von 18-24 C-Atomen, speziell Gammalinolen-, Stearidon-, Omega-9-Eicosatrien (Meadsäure)-, Omega-5-Eicosatrien (Sciadonsäure)-, Dihomogammalinolen-, Arachidon-, Omega-3-Eicosapentaen-, Omega-3-Heneicosapentaen-, Docosatetraen-, Omega-5-docosapentaen-, Omega-6-Docosapentaen (Osbondsäure)- oder Omega-3-Docosahexaensäure; mit einem der folgenden biogenen Resten R':
A) Amine: 1,4-Diaminobutan (Orn), 1,5-Diaminopentan (Lys); Agmatin (Arg), Tryptamin (Trp), Histamin (His), Pyrrolidin (Pro), 3-Hydroxypyrrolidin (Hypro), Isobutylamin (Val), Isoamylamin (Leu), 2-Methylbutylamin (Ileu), Cystamin (CyS-SCy), 2-Hydroxy-1,5-Diaminopentan (Hylys), 3-(Methylthio)-Propylamin (Met).
B) Alkohole: Isobutanol (Val), Isopentanol (Leu), 2-Methylbutanol (Ileu), 1,2-Propandiol (Thr), Tryptophol (Try), Phenylethanol (Phe), 4-Hydroxyphenylethanol (Tyr), 4-Aminobutanol (Orn), 5-Aminopentanol (Lys), 5-Amino-4-Hydroxypentanol (Hylys), 3-(Methylthio)-Propanol (Met), 2-Mercaptoethanol (Cys), Tetrahydrofuran-3-ol (Hypro), 4-Guanidylbutanol (Arg) sowie 2-Imidazolylethanol (His).

Als Derivate der oben beschrieben Substanzgruppen kommen Reste R' der folgenden Art in Frage:
A) von biogenen Aminen: 5-Methoxytryptamin (Trp), Noradrenalin (Tyr), 1,6-Diaminohexan (Lys), 1,7-Diaminoheptan (Lys), 1,8-Diaminooktan (Lys), N-(3-Aminopropyl)-1,4-Diaminobutan (Orn), 1,4-Bis-(3-Aminopropylamino)-Butan (Orn), ), N-(3-Aminopropyl)-1,4-Diaminopentan (Lys), 1,4-Bis-(3-Aminopropylamino)-pentan (Lys), Noragmatin (Arg), 3-Methoxypyrrolidin (Hypro), 2-Triethanolamin (Ala), Morpholinoethylamin (Ala) 3-Hydroxypiperidin (Hypro),
B) von biogenen Alkoholen: 5-Methoxytryptophol (Try), 3,4-Dihydroxyphenylethanol (Tyr), 2-(N-Morpholino)-Ethanol (Ala), 4-Methoxyphenylethanol (Phe), Diethylenglycol (Ala), Diethylenglycolmonomethylether (Ala).

Auch acylierte Derivate der vorstehend genannten Verbindungen sind wirksam, da sie im Körper zu oben genannten Verbindungen hydrolysieren können.

Weiterhin wurde gefunden, dass sich die pharmakologische Wirkung der Aktivsubstanz am CB-1 Rezeptor auch dann voll entfalten kann, wenn diese aktive Wirksubstanz in Mischung mit anderen freien Fettsäuren oder deren Estern im Präparat vorliegt. Überraschenderweise hat sich sogar gezeigt, dass die an den Rezeptoren an sich unwirksamen Begleitstoffe die Wirkung der Aktivsubstanzen zum einen nicht stören, und zum andern sogar die gewünschte Wirkung unterstützen, indem sie nämlich das korrespondierende Abbauenzyms "FAAH" kompetitiv hemmen.

In allen pharmazeutischen Präparaten finden sich abhängig vom jeweiligen Rest R' in der Biomolekel starke agonistische oder antagonistische Wirkungen am CB-1, CB-2 bzw. VR-1 Rezeptor.
Alle Substanzen zeigen erheblich stärkere pharmakologische Wirkungen als das Arachidonsäure-2-Hydroxyethylamid (AEA). Gleichzeitig sind in allen Anwendungsfällen pharmazeutischer Präparate keine störende Nebenwirkungen zu verzeichnen. Dadurch eröffnet sich ein breites Feld von therapeutischen Anwendungsmöglichkeiten für die erfindungsgemäß neu aufgefundenen Stoffe.
Diese Möglichkeiten betreffen folgende pharmakologische Wirkstoffklassen:
Psychopharmaka, Antidepressiva, Anxiolytika, Analgetika, Spasmolytika, Antiemetika, Sedativa, Antihypertensiva, Antiasthmatika, Migränemittel sowie Immunsupressiva. Ferner sind diese Arzneimittel einzusetzen bei motorischen Störungen, zur Reduzierung von L-DOPA Dyskinesien (z.B. bei Parkinsonpatienten), zur Verringerung von Chemotherapie-Nebenwirkungen, zur Glaukombehandlung, als Neuroprotektiva bei Gehirntraumen, bei peripheren vasculären Erkrankungen, zur Appetitanregung oder Appetitunterdrückung, oder sogar zur Förderung von Haarwuchs.

Mit Rücksicht auf den analogen Mechanismus sowohl bei der Darstellung als auch bei der biochemischen Spaltung entsprechender Säurederivate (Amid oder Ester), sowie im Hinblick auf die analoge Elektronenstruktur der -NH-R' und der -O-R' Gruppe kann man viele gemeinsame präparative und pharmakologische Eigenschaften, welche sowohl Entstehung als auch den Metabolismus der Wirksubstanzen betreffen, beobachten. Auch die endogenen CB-1 Liganden liegen teils als Ester und teils als Amide vor. Hingegen sind die Thioester -S-R' im allgemeinen unwirksam.

Bei der Inkorporation dieser biogenen Fettsäurederivate waren in keinem Fall störende Nebenwirkungen im Körper zu beobachten. Je nach Design des Restes R' ließ sich eine pharmazeutische oder therapeutische Anwendung der biogenen Amide oder Ester auch gezielt optimieren. Alle Stoffe können in gewöhnlichen pharmazeutischen Prozessen zu pharmazeutischen Präparaten bzw. Arzneien verarbeitet werden.

Die Darstellung der erfindungsgemäßen Substanzen kann bekanntermaßen auf folgenden Wegen realisiert werden:
A) Umsetzung von entsprechenden Fettsäurechloriden mit primären Aminen oder Alkoholen, bei Ausschluss von Sauerstoff, und Temperaturen unter 100° C.
   Anmerkung: Die Fettsäurechloride werden durch Umsetzung kommerzieller Fettsäuren mit Oxalylchlorid (in CH₂Cl₂) auf schonende Weise, d.h. bei Raumtemperatur, hergestellt.
B) Umsetzung von entsprechenden Fettsäureestern (z.B. natürliche Fette/Öle) mit primären Aminen oder Alkoholen, bei Ausschluss von Sauerstoff und Temperaturen bis 200° C.
   Interessanterweise stört die Anwesenheit weiterer gesättigter oder ungesättigter Fettsäuren nicht. Es gelingt sogar, *natürliche* Fette und/oder Öle mit den entsprechenden Alkoholen bzw. Aminen umzusetzen. Für die weitere pharmakologische Anwendung ist es nicht entscheidend, die tatsächlich wirksamen Verbindungen gesondert zu isolieren.

Es versteht sich für den Fachmann, dass Amide und Ester natürlich auch durch eine Reihe anderer literaturbekannter Synthesewege dargestellt werden können.
Alle neu gefundenen Fettsäurederivate sind offensichtlich in der Lage, die Bluthirnschranke ausreichend zu passieren, um dann insbesondere an den CB-1 Rezeptor zu binden. Es versteht sich, dass eine Vielzahl von Substanzen, die am CB-1 Rezeptor binden auch mit CB-2- und/oder Vanilloid-Rezeptoren (z.B. VR-1) wechselwirken können.

### Experimenteller Teil:

Abkürzungsliste:
Ala = Alanin
Arg = Arginin
Cys = Cystein
CyS-SCy = Cystin
His = Histidin
Hylys = Hydroxy-lysin
Hypro = Hydroxy-prolin
Ileu = Isoleucin
Leu = Leucin
Lys = Lysin
Met = Methionin
Orn = Ornithin
Phe = Phenylalanin
Pro = Prolin
Ser = Serin
Thr = Threonin
Try = Tryptophan
Val = Valin

Folgende Polyensäuren waren Grundlage für die von uns dargestellten neuen Pharmaka:
Gammalinolensäure (GLA), Stearidonsäure (STA), Dihomogammalinolensäure (DGLA), Arachidonsäure (ARA), Eicosapentaensäure (EPA), Heneicosapentaensäure (HPA), Docosatetraensäure (DTA), Docosapentaensäure (DPA) und Docosahexaensäure (DHA).
Es versteht sich für den Fachmann, dass auch Substitutionen an der n-Alkylkette dieser Säuren zu pharmakologisch aktiven Produkten führen können.

Folgende Pharmaka wurden hergestellt:
1) Amide: Durch Umsetzung von biogenen Aminen - die sich durch Decarboxylierung aus den entsprechenden natürlichen Aminosäuren ergeben - mit Fettsäurechloriden, nach folgendem Schema:
   Fettsäurechlorid + biogenes Amin → HCl + biogenes Amid (Produkt)
   R-CO-CI + H₂N-AS → R-CO-NH-AS + HCl (mit ―NH-AS =Y)

### a) 4-Aminobutylarachidonoylamid (1):

Mit dem biogenen Amin: 1,4-Diaminobutan (dem Decarboxylierungsprodukt der natürlichen Aminosäure Ornithin (Orn).

Zunächst haben wir das Säurechlorid der freien Arachidonsäure hergestellt. Die chemische Darstellung des Produkts a) aus dem Säurechlorid wurde dann ausgeführt nach folgender Reaktionsgleichung: 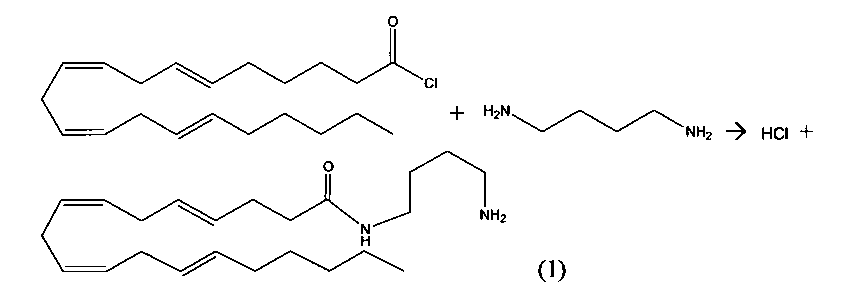
A) Darstellung des Fettsäurechlorids der beispielhaft gewählten Arachidonsäure: 2 mmol Arachidonsäure werden mit 1mmol Oxalylchlorid (in 50 ml Methylenchlorid gelöst) 2 Stunden gerührt und die ausgefallene Oxalsäure abfiltriert. Das dadurch entstandene Arachidonsäurechlorid kann wie folgt direkt weiter verwendet werden:
B) Darstellung eines biogenen Amids der Arachidonsäure:
   Es werden 1 mmol Arachidonsäurechlorid mit 1mmol 1,4-Diaminobutan (in 50 ml Tetrahydrofuran gelöst) bei Raumtemperatur 12 h unter Schutzgasatmosphäre gerührt. Es entsteht das 4-Aminobutylarachidonamid (1). Das (1) enthaltende Produktgemisch wird über reverse phase HPLC gereinigt. Ausbeute an (1) : 81%.
   Das so gewonnene Produkt (1) wird mit handelsüblichen Emulgatoren zu einer Creme mit 10% Wirkstoffgehalt verarbeitet. Von der Creme appliziert man 1 g transdermal. Die Substanz hat deutlich antidepressive und anticonvulsive Wirkungen, ohne dass starke psychotrope Nebenwirkungen konventioneller Cannabinoide auftreten.

### b) (3-Methylthiopropyl)-arachidonoylamid (2):

Mit dem biogenen Amin (3-Methylthiopropyl)-amin, dem Decarboxylierungsprodukt der natürlichen Aminosäure Methionin. (Met)
1) Darstellung des biogenen Amins aus der natürlichen Aminosäure L-Methionin (Met). 4 mmol L-Methionin werden unter Argonschutzgas bei ca. 300° C trocken destilliert. Das Destillationsgemisch wird durch präparative HPLC gereinigt. Es entsteht das biogene Amin: (3-Methylthiopropyl)-amin (in 72% iger Ausbeute).
2) Darstellung des biogenen Amids:
1mmol Arachidonsäurechlorid werden mit 1mmol (3-Methylthiopropyl)-amin (in 50 ml Tetrahydrofuran gelöst) bei Raumtemperatur 12 h unter Schutzgasatmosphäre gerührt. Es entsteht das (3-Methylthiopropyl)-arachidonamid (2). Das Rohprodukt wird über reverse phase HPLC isoliert (in 91% iger Ausbeute).
50 mg des so gewonnenen Reinprodukts (2) wird in handelsübliche Gelatinekapseln gefüllt und oral appliziert.
Neben den bekannten cannabinoiden Wirkungen zeichnet sich dieses Produkt auch durch Wirksamkeit am VR-1 Rezeptor aus, wodurch zusätzlich stimulante bis erotisierende Wirkungen auftreten.

### c) Omega-5-Docosapentaenoyl-(3-Hydroxy)-pyrrolidid (3):

Mit 3-Hydroxypyrollidin, Decarboxylierungsprodukt der natürlichen Aminosäure L-Hydroxyprolin (Hypro).
1) Darstellung des biogenen Amins aus der natürlichen Aminosäure L-Hyroxyprolin (Hypro):
   4 mmol L-Hydroxyprolin werden unter Argonschutzgas bei ca. 300° C trocken destilliert. Das Destillat wird durch präparative HPLC gereinigt. Es entsteht 3-Hydroxypyrollidin (in 72% iger Ausbeute).
2) Darstellung des biogenen Amids (3):
   1mmol Omega-5-Docosapentaensäurechlorid werden mit 1mmol 3-Hydroxypyrollidin (in 50 ml Tetrahydrofuran gelöst) bei Zimmertemperatur 12 h gerührt. Es entsteht das biogene Amid (3).
   Dieses Rohprodukt haben wir erneut über reverse phase HPLC gereinigt (in 71% iger Ausbeute). Anschließend haben wir eine 10%-ige Lösung des Reinprodukts (3) in raffiniertem Sesamöl hergestellt.
   Bereits 20 mg der Substanz (3) transdermal appliziert wirken über 10 Stunden hinweg antidepressiv, analgetisch und motivationsfördernd, ohne dass hypotonische Effekte auftreten, wie man sie beispielsweise von 2-AG bzw. davon abgeleiteten Verbindungen kennt.

### d) Agmatinamide (4): aus Lachsöl

Darstellung biogener Amide (4), direkt aus dem biogenen Amin Agmatin (Agm), dem Decarboxylierungsprodukt der natürlichen Aminosäure L-Arginin (Arg):
1 mmol Agmatin (5-Guanidinylpentylamin), wird mit 2g natürlichem pharmazeutischem Lachsöl (bestehend aus mindestens 18% Eicosapentaensäure (EPA) und mindestens 12% Docosahexaensäure (DHA) neben Docosatetraensäure (DTA) und
Docosapentaensäure (DPA)), bei 200 °C unter Schutzgas 12 h lang gerührt. Es entstehen u. a. die Agamatinylamide (4) der entsprechenden Fettsäuren.
Je 0,5 g dieses Produktgemisches werden in Suppositiorien überführt und rektal appliziert.
Die Arznei zeigt deutliche antidepressive und analgetische Wirkungen. Die Substanz wirkt des weiteren stimmungsaufhellend bis euphorisch, antriebsfördernd und entspannend, ohne die starken psychotropen Nebenwirkungen konventioneller Cannabinoide. Es treten keinerlei merkbare störende Nebenwirkungen auf.

### 2) Biologisch aktive Amide aus Derivaten von folgenden biogenen Aminen:

1,6-Diaminohexan vom Cadaverin, dem biogenen Amin des Lysins (Lys), N-(3-Aminopropyl)-1,4-Diaminobutan vom biogenen Amin des Ornithins. (Orn), Triethanolamin vom biogenen Amin des Serins (Ser), 2-Morpholinoethylamin vom biogenen Amin des Alanins (Ala).

Weitere von biogenen Aminen ableitbare Verbindungen sind z.B.
1,7- Diaminoheptan, 1,8-Diaminooktan, (Kettenverlängerungsprodukte von Cadaverin, dem biogenen Amin des Lysins (Lys)), 1,4-Bis-(3-Aminopropyl)-amino-butan, vom biogenen Amin des Ornithins (Orn), N-(3-Aminopropyl)-1,4-Diamino-pentan vom biogenen Amin des Lysins (Lys), 1,4-Bis-(3-Aminopropyl)-amino-pentan, vom biogenen Amin des Lysins (Lys), Noragmatin von Agmatin (Arg), 3-Hydroxypiperidin von 3-Hydroxypyrollidin (Hypro).

### a) EPA-(6-aminohexyl)-amid (5):

1mmol Omega-6-Eicosapentaensäurechlorid werden mit 1mmol 1,6-Diaminohexan (in 50 ml Tetrahydrofuran gelöst) bei Raumtemperatur unter Schutzgas 6 h gerührt. Das Produktgemisch wird über reverse phase HPLC gereinigt. In Ausbeute von 71% entsteht (5).
Ein pharmazeutisches Präparat, in welchem ca. 30 mg aktive Substanz (5) enthalten ist, zeigt ähnliche Wirkeffekte wie 4-Aminobutyl-arachidonoylamid (s. 1 a).
Störende Nebenwirkungen sind hierbei ebenfalls in keiner Weise zu Tage getreten. Wir gehen davon aus, dass beim Metabolismus dieser Substanzklasse sehr wenig toxische Amine entstehen, welche von körpereigenen Enzymen problemlos weiter degradiert werden können. Die von uns neu synthetisierten 6-Aminohexylamide zeigen keine Wirkung an VR-1 Rezeptoren.

### b) DPA-N-(3-Aminopropyl)-4-aminobutylamid (6): aus Pinienkernen

### 1) Darstellung des Fettsäurechlorids:

Aus natürlichen Pinienkernen wird das fette Öl durch Extraktion mit THF isoliert und mit entsprechenden Mengen NaOH vollständig hydrolysiert. Aus den Fettsäuren des Hydrolysegemisches wird die Omega-5-Docosapentaensäure durch reverse phase HPLC abgetrennt und mittels Oxalylchlorid in das Omega-5-Docosapentaensäurechlorid überführt.
2) Amidisierung mit dem biogenen Amin N-(3-Aminopropyl)-1,4-Diaminobutan (Orn):
1 mmol Omega-5-Docosapentaensäurechlorid, wird mit 1mmol N-(3-Aminopropyl)-1,4-Diaminobutan (in 50 ml Tetramethylethylendiamin (TMEDA) gelöst) bei Zimmertemperatur unter Schutzgas 3 h gerührt. Hierbei wird die freiwerdende HCl durch das basische Lösungsmittel gebunden. Die Endprodukte werden wieder über reverse phase HPLC isoliert. Es entstehen vorwiegend N-(3-Aminopropyl)-4-Aminobutyl-Docosapentaenylamid (6), neben N-(4-Aminobutyl)-3-aminopropyldocosapentaenylamid in Ausbeuten >55 % bzw. >30 %.
Beide Produkte sind pharmakologisch in ähnlicher Wiese aktiv, so dass an sich zur Herstellung eines pharmazeutischen Präparats keine Trennoperation erforderlich ist. Die entstehenden Produkte sind hervorragend resorbierbar und können leicht mit "Carriers", wie z.B. Sesamöl oder DMSO durch die intakte Haut oder Schleimhäute in die Blutbahn transportiert werden.
Das Produkt wirkt über mehr als 6 Stunden hinweg stimmungsaufhellend bis euphorisch, antriebsfördernd und entspannend, ohne dass cannabisähnliche psychotrope Wirkungen auftreten. Hypotonische Effekte wie bei 2-AG, oder VR-1 agonistische Wirkungen, wie bei Arachidonsäure-2-hydroxyethylamid (AEA), wurden ebenfalls nicht festgestellt.

### c) Bis-(2-Hydroxyethyl)-Virodhamin (7):

1mmol Eicosapentaensäurechlorid (z.B. gewinnbar aus Fischölen) wird -zur Vermeidung von Mehrfachacylierungen- mit 10 mmol Triethanolamin unter Argon 3 Stunden bei Raumtemperatur gerührt. Es entsteht das gewünschte Produkt Bis-(2-hydroxyethyl)-Virodhamin (7), neben Triethanolammoniumchlorid. Das gewünschte Produkt (7) wird mit Essigsäureethylester hinreichend rein aus der wässrigen Phase extrahiert.
Ein Teil des Produkts (7) wird mit geringem Überschuss Acetanhydrid zum Mono- bzw. Diacetylderivat acetyliert. Es zeigt sich nämlich, dass alle drei neuen Derivate pharmakologisch aktiv sind.
Zur Darstellung eines pharmazeutischen Präparats haben dann wir im Folgenden eingesetzt:
a) Bis-(2-hydroxyethyl)-virodhamin (7).
b) Das Monoacetylderivat des Bis-(2-hydroxyethyl)-virodhamins (8).
c) Das Diacetylderivat des Bis-(2-hydroxyethyl)-virodhamins (9).
Ein pharmazeutisches Präparat in welchem ca. 30 mg aktive Substanz (7) enthalten war, zeigt sedative Wirkungen und das typische Profil eines CB-1 Agonisten. (Euphorie, Entspannung, Appetitanregung, Schmerzlinderung). Störende Nebenwirkungen sind hierbei ebenfalls in keiner Weise zu verzeichnen. Beim Metabolismus dieser Substanzklasse und ihrer Acylderivate entstehen praktisch ausschließlich non-toxische Abbauprodukte, die von körpereigenen Enzymen problemlos weiter zerlegt werden.
Es zeigte sich zudem, dass das Mono-, insbesondere aber das Diacetylderivat des Bis-(2-hydroxyethyl)-virodhamins (8) bzw. (9) deutlich schneller und intensiver wirkten als das nicht acetylierte Produkt (7). Daneben war im Gegensatz zum freien Bis-(2-hydroxyethyl)-virodhamins (7) eine eher stimulierende Wirkung zu beobachten.
Erst kürzlich wurde gezeigt, dass unsubstituiertes Virodhamin (Arachidonsäure-2-aminoethylester) im Säugetiergehirn ein endogener CB-1 *Antagonist* ist. Umso überraschender war unsere Feststellung, dass das Bis-2-Hydroxyethylderivat des Virodhamins (7) massiv *agonistische* Effekte am CB-1 Rezeptor entfaltet.

### d) Arachidonoyl-(2-Morpholino)-ethylamid (10):

1 mmol Arachidonsäurechlorid werden mit 1mmol 2-Morpholinoethylamin (in 50 ml Tetrahydrofuran gelöst) bei Raumtemperatur unter Schutzgas 12 h gerührt.
Es bildet sich das gewünschte Arachidonoyl-(2-Morpholino)-ethylamid (10). Das Produktgemisch wird über reverse phase HPLC gereinigt. In Ausbeute von 89% entsteht (10).
Je 40 mg des so hergestellten Produkts (10) werden in handelsübliche Gelatinekapseln gefüllt und oral appliziert. Dieses Produkt wird im Verdauungstrakt rasch resorbiert und erzeugt typische cannabinoide Wirkungen.

### 3) Ester: die sich sich von folgenden biogenen Alkoholen ableiten lassen.

Sie entstehen aus den entsprechenden biogenen Aminen durch Substitution der NH₂ Gruppe durch die OH Gruppe am Aminosäuregrundkörper. Es wurden Ester synthetisiert von: Isobutanol (Val), Isopentanol (Leu), 2-Methylbutanol (Ileu), 1,2-Propandiol (Thr), Tryptophol (Try), 2-Phenylethanol (Phe), 4-Hydroxyphenylethanol (Tyr), 5-Aminopentanol (Lys), 5-Amino-4-Hydroxypentanol (Hylys), 3-Methylthiopropanol (Met), 2-Mercaptoethanol (Cys), 4-Guanidylbutanol (Arg) und 2-Imidazolylethanol (His).
Alle Ester der genannten biogenen Alkohole mit den entsprechenden Polyensäuren führen überraschenderweise ebenfalls zu Produkten mit pharmakologischer Aktivität; vergleichbar mit den vorstehend genannten Aminen. Auch hier findet man, dass die Aktivsubstanzen in der Zelle zu biokompatiblen Abbauprodukten degradiert werden, so dass auch von diesen Strukturen keine signifikanten Nebenwirkungen zu erwarten sind. Zur Darstellung der folgenden Ausführungsbeispiele sind die Säurechloride entsprechender Fettsäuren, wie bereits bei den Aminen gezeigt, eingesetzt worden.

### a) Arachidonsäure-2-Phenylethylester (11):

1mmol Arachidonsäurechlorid (gewinnbar aus z.B. Schweinefett oder ARASCO®-öl) wird mit 2 mmol des biogenen Alkohols 2-Phenylethanol (Phe) in Gegenwart von 2 mmol Triethylamin als Hilfsbase unter Argon 4 Stunden bei Raumtemperatur gerührt. Es entsteht das Produkt 2-Phenylethylarachidonat (11) neben Triethylammoniumchlorid. Das Produkt wird mit Ethylacetat hinreichend rein aus der wässrigen Phase extrahiert. Dieses Rohprodukt (11) haben wir direkt mit der fünffachen Menge 80% Dimethylsulfoxid (DMSO) verrührt. Bereits 50 mg der Substanz (3) mit DMSO transdermal appliziert zeigten das typische Profil cannabinoider Wirkstoffe (antidepressiv, anxiolytisch, analgetisch, spasmolytisch, hypothermieerzeugend).

### 4) Ester: die sich von einfachen Derivaten biogener Alkohole ableiten lassen:

Wirksame Derivate biogener Alkohole sind:
5-Methoxytryptophol (Try), 3,4-Dihydroxyphenylethanol (Tyr), 2-(N-Morpholino)-ethanols (Ala), 4-Methoxyphenylethanol (Phe), Diethylenglycol (Ala), Diethylenglycolmonomethylether (Ala).
Die Fettsäureester der unter 4) aufgeführten Alkohole zeigten, dass auch längerkettige und unverzweigte Ester der einschlägigen Polyensäuren an CB-Rezeptoren aktiv sein können. Es ist anzunehmen, dass auch hier die Aktivsubstanzen in der Zelle zu nebenwirkungsarmen Abbauprodukten degradiert werden.

### a) Arachidonsäure-2-(N-Morpholino)-ethylester (12):

Analog zur Synthese von Arachidonoyl-2-(N-Morpholino)-ethylamid (10) wird der Arachidonsäureester des 2-(N-Morpholino)-ethanols (12) dargestellt.
Ein pharmazeutisches Präparat mit der Ester-Wirksubstanz (12) zeigt ähnliche pharmakologische Effekte wie das Amid (10), allerdings von kürzerer Dauer.

Anmerkung: Ein 2-(N-Morpholino)-Ethylfettsäureester geht auch durch intramolekulare Wasserabspaltung zwischen zwei Hydroxylresten im Triethanolamin hervor, sofern beim Herstellungsprozesses von (7) Temperaturen von über 240 °C auftreten.

Es ist möglich, die neu gefundenen Substanzen in Reinform oder Mischpräparaten aller Wirksubstanzen (z.B. (1) bis (12)) pharmazeutisch und gezielt therapeutisch zu designen und einzusetzen.

### Literatur:

1) Devane, W. A., Hanus, L., Breuer, A., Pertwee, R. G., Stevenson, L. A., Griffin, G.,Gibson, D. and Mechoulam, R. (1992) Isolation and structure of a brain constituent that binds to the cannabinoid receptor. Science 258, 19461949
2) Mechoulam et al. US 5,618,955
3) J. Med. Chem. 1997 Oct 24; 40(22): 3617-25
4) Mechoulam et.al. "Identification of an endogenous 2-monoglyceride..." *Biochem Pharmacol* 50 (1995) 83-90.
5) S. Munro et. al. Nature, 365, 61-65, 1993
6) M. J. Caterina et. al. "The capsaicin receptor..." Nature 389, 816-824 (1997)
7) Di-Marzo et. Al. Eur-J-Pharmacol. 2001 May 25; 420(2-3): 123-31
8) Strelchenok US 6,403,554 (2002).
9) Tognetto,-M; Amadesi,-S et. al. J. Neurosci. 2001 Feb 15; 21 (4): 1104-9

## Patentansprüche

1. Arzneimittel, enthaltend eine Fettsäureverbindung der Formel R-CO-Y mit R als Omega-x-Polyensäurerest mit x=3-9 und einer Gesamtkohlenstoffanzahl zwischen C₁₇ und C₂₃ sowie einer Anzahl an isolierten Doppelbindungen zwischen 3 und 6, und Y als Rest eines biogenen Amins der Struktur -NH-R', wobei das biogene Amin eine decarboxylierte natürliche Aminosäure AS oder ein einfaches Derivat davon ist, **dadurch gekennzeichnet, dass** die natürliche Aminosäure AS aus der Reihe Val, Ileu, Leu, Pro, Hypro, Arg, CyS-SCy, Met, Lys, Hylys, Orn, Trp, His stammt und wodurch als Wirksubstanz ein entsprechendes Fettsäureamid ausgebildet wird.

2. Arzneimittel, enthaltend eine Verbindung der Formel R-CO-Y mit R als Omega-x-Polyensäurerest mit x=3-9 und einer Gesamtkohlenstoffanzahl zwischen C₁₇ und C₂₃ sowie einer Anzahl an isolierten Doppelbindungen zwischen 3 und 6, und Y als Rest eines Amins der Struktur -NH-R', wobei das Amin ein einfaches Derivat einer decarboxylierten natürlichen Aminosäure AS ist, **dadurch gekennzeichnet, dass** die natürliche Aminosäure AS aus der Reihe Ala, Ser, Thr, Phe, Tyr, Cys stammt und wodurch als Wirksubstanz ein entsprechendes Fettsäureamid ausgebildet wird.

3. Arzneimittel, enthaltend eine Verbindung der Formel R-CO-Y mit R als Omega-x-Polyensäurerest mit x=3-9 und einer Gesamtkohlenstoffanzahl zwischen C₁₇ und C₂₃ sowie einer Anzahl an isolierten Doppelbindungen zwischen 3 und 6, und Y als Rest eines biogenen Alkohols der Struktur ―O-R' oder eines einfachen Derivates davon, wobei der biogene Alkohol von einer decarboxylierten natürlichen Aminosäure AS dadurch abgeleitet ist, dass deren alpha-Aminogruppe durch eine Hydroxylgruppe ersetzt ist, **dadurch gekennzeichnet, dass** die natürliche Aminosäure AS aus der Reihe Ala, Val, Ileu, Leu, Phe, Hypro, Arg, Thr, Cys, CyS-SCy, Met, Lys, Hylys, Orn, Trp und His stammt und wodurch als Wirksubstanz ein entsprechender Fettsäureester ausgebildet wird.

4. Arzneimittel nach Anspruch 1 bis 3, **dadurch gekennzeichnet, dass** der Omega-x-Polyensäurerest R der Wirksubstanz aus der Gruppe: Gammalinolen-, Stearidon-, Omega-9-Eicosatrien- (Meadsäure), Omega-5-Eicosatrien-(Sciadonsäure), Dihomogammalinolen-, Arachidon-, Omega-3-Eicosapentaen-, Omega-3-Heneicosapentaen-, Omega-9-Docosatetraen-, Omega-5-docosapentaen-, Omega-6-Docosapentaen-, (Osbondsäure), Omega-3-Docosahexaensäure stammt.

5. Arzneimittel nach Anspruch 1 bis 4, **dadurch gekennzeichnet, dass** der Omega-Polyensäurerest R der Wirksubstanz in all-Z-Konformation vorliegt.

6. Arzneimittel nach Anspruch 1 bis 5, **dadurch gekennzeichnet, dass** die dem Polyensäurerest R der Wirksubstanz zugrundeliegende Polyensäure aus einem Naturprodukt, vorzugsweise Borretschöl, Nachtkerzenöl, Johannisbeersamenöl, Hanföl, Pinienkernöl, Fischöle oder Schweinefett, oder aus einem gentechnisch verändertem Mikroorganismus isoliert worden ist.

7. Arzneimittel nach Anspruch 1 bis 6, **dadurch gekennzeichnet, dass** die aktive Wirksubstanz neben anderen ungesättigten oder gesättigten freien Fettsäuren oder deren Estern im Präparat vorliegt oder in Kombination mit anderen wirksamen Pharmaka oder in Kombination mit pflanzlichen Cannabinoiden vorliegt.

8. Arzneimittel nach einem der Ansprüche 1 oder 2 oder 4 bis 7, **dadurch gekennzeichnet, dass** der Aktivteil Y der Wirksubstanz ein Rest eines biogenen Amins aus der Gruppe: Agmatin (Arg), Tryptamin (Trp); Histamin (His), Pyrrolidin (Pro), Hydroxypyrrolidin (Hypro), Isobutylamin (Val), Isoamylamin (Leu), 2-Methylbutylamin (Ileu), Cystamin (CyS-SCy), 1,4-Diaminobutan (Orn), 1,5-Diaminopentan (Lys), 2-Hydroxy-1,5-Diaminopentan (Hylys), 3-Methylthiopropylamin (Met) ist.

9. Arzneimittel nach Anspruch 1 bis 8, **dadurch gekennzeichnet, dass** der Aktivteil Y der Wirksubstanz der Rest eines der folgenden einfachen Derivate biogener Amine ist: 5-Methoxytryptamin (Try), Noradrenalin (Tyr), 1,6-Diaminohexan (Lys), 1,7-Diaminoheptan (Lys), 1,8-Diaminooktan (Lys), N-(3-Aminopropyl)-1,4-Diaminobutan (Orn), 1,4-Bis-(3-Aminopropylamino)-butan (Orn), N-(3-Aminopropyl)-1,4-Diaminopentan (Lys), 1,4-Bis-(3-Aminopropylamino)-pentan (Lys), Noragmatin (Arg), Triethanolamin (Ser), 3-Hydroxypiperidin (Hypro), 2-Morpholinoethylamin (Ala).

10. Arzneimittel nach Anspruch 3 bis 9, **dadurch gekennzeichnet, dass** der Aktivteil Y der Wirksubstanz ein Rest eines biogenen Alkohols aus der Gruppe: Isobutanol (Val), Isopentanol (Leu), 2-Methylbutanol (Ileu), 1,2-Propandiol (Thr), Tryptophol (Try), Phenylethanol (Phe), 4-Hydroxyphenylethanol (Tyr), 4-Aminobutanol (Orn), 5-Aminopentanol (Lys), 5-Amino-4-hydroxypentanol (Hylys), Tetrahydrofuran-3-ol (Hypro), 3-Methylthiopropanol (Met), 2-Mercaptoethanol (Cys), 4-Guanidylbutanol (Arg), 2-Imidazolylethanol (His) ist.

11. Arzneimittel nach Anspruch 3 bis 10, **dadurch gekennzeichnet, dass** der Aktivteil Y der Wirksubstanz der Rest eines der folgenden einfachen Derivate biogener Alkohole ist: 5-Methoxytryptophol (Try), 3,4-Dihydroxyphenylethanol (Tyr), 2-(N-Morpholino)-Ethanols (Ala), 4-Methoxyphenylethanol (Phe), Diethylenglycol (Ala), Diethylenglycolmonomethylether (Ala).

12. Verfahren zur Herstellung der Wirksubstanz eines Arzneimittels nach einem der vorhergehenden Ansprüche, **dadurch gekennzeichnet, dass** in einem ersten Verfahrensschritt das eingesetzte Amin H₂N-R' oder der eingesetzte Alkohol HO-R' mit einem Säurechlorid oder einem Ester oder einem Anhydrid oder einem verwandten Derivat der Polyensäuren acyliert wird, in einem zweiten Verfahrensschritt das gewünschte wirksame Reaktionsprodukt aus dem Reaktionsansatz durch gängige physiko-chemische Methoden abgetrennt wird und in einem dritten Verfahrensschritt das abgetrennte Reaktionsprodukt in eine medizinisch applizierbare Form überführt wird.

13. Verfahren zur Herstellung eines Arzneimittels nach einem der vorhergehenden Ansprüche, **dadurch gekennzeichnet, dass** die Wirksubstanz in der Arznei in Konzentrationen von größer 0,1 % oder vorzugsweise in Konzentrationen größer 0, 1 % in Sesamöl vorliegt.

14. Arzneimittel nach einem der vorhergehenden Ansprüche, **dadurch gekennzeichnet, dass** die aktive Wirksubstanz zur Bekämpfung von Krankheiten und Störungen angewendet wird, wie z.B. Depression, Angst, Schmerz, Krampflösung, Erbrechen, Bluthochdruck, psychomotorische Unruhe, Dyskinesie, Überagitiertheit, Schlaflosigkeit, Migräne, Asthma, bestimmte Formen von Schizophrenie, Chemotherapie Nebenwirkungen, erhöhter Augeninnendruck, multiple Sklerose, periphere vasculäre Erkrankungen oder als Immunsupressivum oder als Neuroprotektivum bei Gehirntraumen oder als Inhibitor von Tumor Necrosis Factor (TNF-alpha) bei Carcinomen, oder zur Appetitanregung oder zur Förderung von Haarwuchs oder vorzugsweise zur Bekämpfung von psychischen oder psychosomatischen Krankheiten oder als Schmerzbekämpfungsmittel.
